(19) Europäisches Patentamt
European Patent Office
Office européen des brevets

(11) Veröffentlichungsnummer: **0 163 280**
**A1**

# (12) EUROPÄISCHE PATENTANMELDUNG

(21) Anmeldenummer: 85106466.7

(22) Anmeldetag: 25.05.85

(51) Int. Cl.⁴: C 07 F 9/65
C 07 D 239/36, A 01 N 57/16
A 01 N 57/24

(30) Priorität: 30.05.84 DE 3420166

(43) Veröffentlichungstag der Anmeldung:
04.12.85 Patentblatt 85/49

(84) Benannte Vertragsstaaten:
AT BE CH DE FR GB IT LI NL

(71) Anmelder: BASF Aktiengesellschaft
Carl-Bosch-Strasse 38
D-6700 Ludwigshafen(DE)

(72) Erfinder: Spiegler, Wolfgang, Dr.
Westpreussenstrasse 5
D-6520 Worms 27(DE)

(72) Erfinder: Goetz, Norbert, Dr.
Schoefferstrasse 25
D-6520 Worms 1(DE)

(72) Erfinder: Adolphi, Heinrich, Dr.
Kalmitweg 11
D-6703 Limburgerhof(DE)

(72) Erfinder: Buerstinghaus, Rainer, Dr.
Weinbergstrasse 85
D-6940 Weinheim(DE)

## (54) Pyrimidylphosphorsäureester, Verfahren zu ihrer Herstellung und ihre Verwendung zur Bekämpfung von Schädlingen.

(57) Die Erfindung betrifft neue trihalogenmethylsubstituierte Pyrimidylphosphorsäureester, ihre Herstellung, Vorprodukte dafür, die Ester enthaltende Schädlingsbekämpfungsmittel und die Verwendung der Ester als Schädlingsbekämpfungsmittel.

EP 0 163 280 A1

Pyrimidylphosphorsäureester, Verfahren zu ihrer Herstellung und ihre Verwendung zur Bekämpfung von Schädlingen

Es ist aus der DE-AS 11 56 274 und der DE-OS 30 03 337 bekannt, daß unter den insektizid wirkenden Phosphorverbindungen auch heterocyclisch substituierte Phosphorsäurederivate sind. Sie eignen sich zur Bekämpfung von Insekten und Spinnentieren. Ihre spezifische Wirkung ist jedoch oft gering, so daß Bedarf für Mittel besteht, die bei geringerer Konzentration als die bekannten Mittel wirken.

Es wurde gefunden, daß Phosphorsäurederivate der Formel I

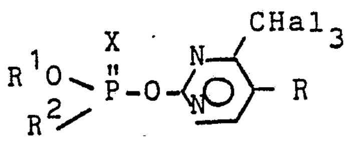 (I),

in der

R Wasserstoff oder Niederalkyl, das gegebenenfalls durch Halogen, Niederalkoxy, -Alkylthio, -Aryloxy oder Aryl substituiert ist,

$R^1$ eine Niederalkyl,

$R^2$ Niederalkoxy- oder -alkylthio, Niederalkyl, Phenyl, Niederalkylamino- oder Niederdialkylamino,

X Sauerstoff oder Schwefel und

Hal Fluor oder Chlor bedeuten,

insektizid, akarizid und nematizid sehr gut wirksam und bekannten Wirkstoffen ähnlicher Struktur bzw. gleicher Wirkungsrichtung überlegen sind.

Die Ester der Formel I können in an sich bekannter Weise durch Umsetzung entsprechender 2-Hydroxy-4-trihalogenmethylpyrimidine mit entsprechenden (Thiono)(Thiol)Phosphor(Phosphon)säureester(amid)-halogeniden erhalten werden:

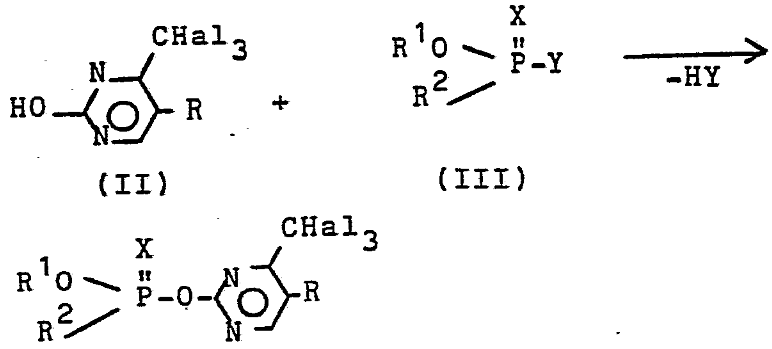


(II) (III)

(I)

Y steht für Halogen und bedeutet aus wirtschaftlichen Gründen vorzugsweise ein Chloratom.

Die Umsetzung wird zweckmäßig in einem Lösungs(Verdünnungs-)mittel und in Gegenwart eines Säureakzeptors vorgenommen. Als Lösungsmittel sind beispielsweise geeignet: aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlormethan, Chlorbenzol, wie Diethyl- und Di-n-butylether, Methyl-tert.-butylether, Tetrahydrofuran, Dioxan; Ketone, beispielsweise Aceton, Methylethylketon, Methylisoproppylketon; ferner Nitrile, wie Acetonitril und Propionitril. Auch Gemische dieser Stoffe können verwendet werden.

Als Säurebindemittel (Säureakzeptoren) eignen sich die bei der Phosphorylierung von heterocyclischen Hydroxyverbindungen üblichen basischen Mittel. Besonders geeignet sind Alkalimetallcarbonate oder -alkoholate, wie Natrium- und Kaliumcarbonat, -methylat und -ethylat, ferner aliphatische, aromatische und heterocyclische Amine, z. B. Triethylamin, Dimethylamin, Piperidin, Dimethylanilin, Dimethylbenzylamin und Pyridin. In einigen Fällen ist die Verwendung von Alkyllithiumverbindungen, z. B. n-Butyllithium oder Alkalimetallhydriden, z. B. Natriumhydrid vorteilhaft.

Anstelle des Zusatzes eines Säureakzeptors kann man auch vor der Umsetzung die Salze der 2-Hydroxy-4-trihalogenmethylpyrimidine etwa die Alkalimetall-, Erdalkalimetall- oder Ammoniumsalze herstellen und diese umsetzen.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung verläuft gewöhnlich oberhalb von Raumtemperatur mit ausreichender Geschwindigkeit. 120 °C müssen i. a. nicht überschritten werden. In einigen Fällen wird so viel Wärme entwickelt, daß es von Vorteil sein kann, eine Kühlmöglichkeit vorzusehen.

Das Reaktionsgemisch wird in üblicher Weise aufgearbeitet, z. B. durch Versetzen mit Wasser, Trennen der Phasen und Destillation und/oder Säulenchromatographie.

Die als Zwischenprodukte benötigten 2-Hydroxy-4-trihalogenmethylpyrimidine (II) sind neue Stoffe. Nach einem im Zusammenhang mit der Erfindung gefundenen Verfahren sind sie aus Trihalogenessigsäureanhydriden oder Trihalogenessigsäurehalogeniden, einem Vinylether und Harnstoff leicht zugänglich.

Hierzu ist folgendes vorauszuschicken.

Es ist bekannt, daß Trihalogencarbonsäureanhydrid bzw. Trichloracetylchlorid (IIa) mit einem Enolether (IIb) zu einem Trihalogenmethyl-ß--alkoxyvinylketon umgesetzt werden kann (Chemistry Letters, 1976, 499; Chemische Berichte 115, 2766 (1982); DE-OS 32 12 136). Diesen Umsetzungsprodukten kommt je nach Reaktionsbedingungen die allgemeine Struktur A oder B zu

$HalC\text{-}CO\text{-}CHR\text{-}CHY\text{-}OR^1$ (A)

$Hal_3C\text{-}CO\text{-}CR{=}CH\text{-}OR^1$ (B),

wobei Hal, Y, R und $R^1$ die obengenannte Bedeutung haben.

Es wurde nun gefunden, daß die Pyrimidine II aus den vorgenannten aus Trichlormethylcarbonsäurehalogeniden oder Trichlormethylcarbonsäureanhydriden (IIa) und Enolethern (IIb) zugänglichen Trihalogenmethylketonen A bzw. B und Harnstoff in hoher Ausbeute erhalten werden:

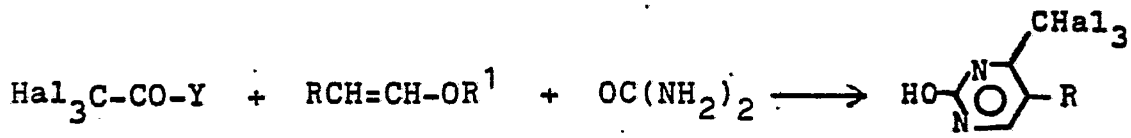

(IIa) (IIb) (II)

Auf eine Isolierung des Anlagerungsproduktes von IIa und IIb kann verzichtet werden, so daß sich die Durchführung der Reaktion besonders einfach gestalten läßt.

Man verfährt zweckmäßig so, daß man zunächst das Trihalogenmethylcarbonsäurederivat (IIa) mit dem Enolether (IIb) umsetzt. Dabei kann entweder IIa zu IIb oder umgekehrt IIb zu IIa zugesetzt werden. Die Reaktion verläuft mit oder bevorzugt ohne Zugabe von Lösungsmitteln.

In Frage kämen als Lösungsmittel Benzol, Toluol, o-, m-, p-Xylol, Chlorbenzol, o-, m-, p-Dichlorbenzol, Nitrobenzol, o-Nitrotoluol, o-, m-, p-Chlornitrobenzol, chlorierte Kohlenwasserstoffe wie z.B. 1,2-Dichlormethan, Trichlormethan, Tetrachlormethan, 1,2-Dichlorethan, 1,1,1-Trichlorethan, 1,1-Dichlorpropan, 1,3-Dichlorpropan, Dialkylether wie z.B. Diethylether, Diisopropylether, tert.-Butylmethylether und cyclische Ether, wie z.B. Tetrahydrofuran, Dioxan oder Gemische aus den genannten Lösungsmitteln.

Die Umsetzung (Anlagerung) von IIa und IIb verläuft an sich spontan; in Gegenwart einer Base, z.B. eines Trialkylamins, eines N,N-Dialkylanilins oder eines gegebenenfalls substituierten Pyridins werden eine gewisse Beschleunigung und auch weniger Nebenprodukte beobachtet. Es kann auch nach der Reaktion von IIa und IIb eine Base zugegeben werden.

Vor der Zugabe des Harnstoffs kann die u.U. im Überschuß vorhandene Base mit einer Mineralsäure, z.B. Salzsäure oder Schwefelsäure neutralisiert und das Gemisch darüber hinaus angesäuert werden.

Die Umsetzung mit Harnstoff wird zweckmäßig so durchgeführt, daß man eine Lösung des Harnstoffs in einem Wasser-Alkohol-Gemisch zum Umsetzungsprodukt von IIa und IIb zugibt. Es ist aber auch möglich, zunächst nur Wasser oder Alkohol und dann Harnstoff in fester Form zuzugeben.

Das Umsetzungsprodukt von IIa und IIb kann als Zwischenprodukt vor der Umsetzung mit Harnstoff auf bekannte Weise isoliert werden; Vorteile bietet diese zusätzliche Maßnahme aber nicht.

Die Umsetzung (Anlagerung) von IIa und IIb verläuft schon unter 0°C und benötigt i.a. keine Temperatur oberhalb von 100°C. Die Umsetzung mit Harnstoff erfolgt zweckmäßig zwischen 20°C und 100°C. Beide Teilschritte sind aber auch gegen höhere Temperaturen nicht sehr empfindlich. Bei Bedarf kann das Verfahren fortlaufend gestaltet werden.

Die Reaktionszeiten betragen bei geeigneten Temperaturen i.a. nicht mehr als 6 Stunden. Nach dem Abkühlen fällt das Produkt in fester Form an und kann abgesaugt werden.

Die nach dem erfindungsgemäßen Verfahren hergestellten neuen 2-Hydroxypyrimidine werden als Zwischenprodukte für die Herstellung der eingangs beschriebenen Phosphorsäureester benötigt. Sie können außerdem z.B. in üblicher Weise (A. Weissberger, The Chemistry of Heterocyclic Compounds, Intersience Publishers, 1962, Seite 162 und Supplement 1, 1970, Seite 110) einfach in die entsprechenden 2-Chlorverbindungen (IIc)

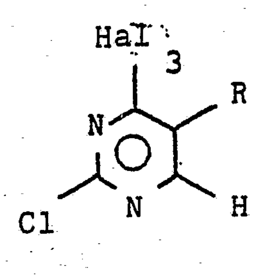

(IIc)

überführt werden, die sowohl als Zwischenprodukte (GB-PS 910 125) als auch als Pflanzenschutzmittel (Jap.PS 28 657; GB-PS 1 013 460) von Interesse sind.

Die zur Synthese der Verbindungen der Formel I außerdem benötigten Phosphorsäureesterhalogenide III sind aus Houben-Weyl, Methoden der organischen Chemie, 4. Auflage, Band XII/2, S. 274 ff, Stuttgart (1964) bekannt und lassen sich auf den dort beschriebenen Synthesewegen herstellen.

Die Wirkstoffe (Ester) der Formel I fallen teilweise in Form farbloser oder schwach bräunlich gefärbter Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisation erfolgen.

Zur Charakterisierung der neuen Phosphorsäurederivate der Formel I dienen im folgenden H-NMR-Spektren, Elementaranalyse und IR-Spektren mit typischen Absorptionsmaxima aus dem sog. "fingerprint"-Bereich zwischen 1 500 $cm^{-1}$ und 900 $cm^{-1}$.

Die folgenden Beispiele 1 und 2 erläutern die Herstellung der Phosphorsäureester der Formel I:

Beispiel 1

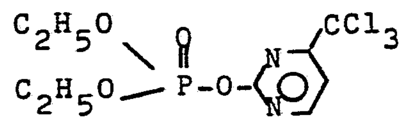

15,98 g 2-Hydroxy-4-trichlormethylpyrimidin werden in 180 ml Methanol suspendiert. Unter Rühren fügt man 13,5 g einer 30 %igen Natriummethylatlösung hinzu und zieht das Methanol wieder vollständig ab. Das als hellgelber Feststoff anfallende Natriumsalz wird in 150 ml Dimethylformamid gelöst und tropfenweise mit 13,57 g Phosphorsäure-O,O-diethylesterchlorid versetzt. Man läßt 5 Stunden bei 55 °C nachrühren, gießt in das dreifache Volumen Eiswasser und extrahiert wiederholt mit Methyl-tert.-butylether. Man wäscht mit Wasser, trocknet über Natriumsulfat und entfernt den Ether. Es verbleiben 15,9 g O,O-Diethyl-O-(4-trichlormethyl-2-pyrimidinyl)-phosphat als ölige Flüssigkeit.
Ausbeute: 61 % der rechnerisch möglichen.
$C_9H_{12}Cl_3N_2O_4P$ (349,5)
ber.: C 30,9 H 3,5 N 8,0
gef.: C 31,1 H 3,4 N 8,1
Infrarotabsorption ($cm^{-1}$): 1431, 1387, 1284, 1166, 1064, 1027, 991, 966, 897.

Beispiel 2

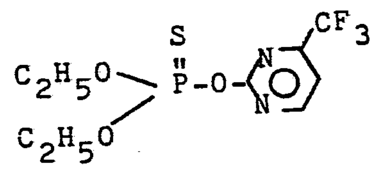

0,95 g einer 80 %igen Suspension von Natriumhydrid in Paraffinöl werden mit 5 ml Dimethylformamid versetzt. Unter Rühren setzt man eine Lösung von 4,92 g 2-Hydroxy4-trifluormethylpyrimidin in 20 ml Dimethylformamid

nach und nach zu. Die Umsetzung erwärmt sich und setzt Wasserstoff frei. 3 Stunden später werden 5,65 g Thiophosphorsäure-O,O-diethylesterchlorid hinzugefügt und danach 12 Stunden bei 25 °C gerührt. Man gießt in die fünffache Menge Wasser und extrahiert mehrfach mit Methyl-tert.-butylether. Man wäscht mit Wasser und trocknet über Natriumsulfat. Nach Entfernen des Lösungsmittels, zuletzt bei 50 °C und 0,01 mbar, erhält man 6,5 g O,O-Diethyl-O-(4-trifluormethyl-2-pyrimidinyl)-phosphothioat als schwachbraune Flüssigkeit.
Ausbeute: 70 % der rechnerisch möglichen.
$C_9H_{12}F_3N_2O_3PS$ (316)
ber.: C 34,2 H 3,8 N 8,9
gef.: C 34,2 H 4,0 N 9,0
Infrarotabsorptionen ($cm^{-1}$): 1388, 1341, 1194, 1160, 1140, 1021, 995, 966.

Die nachstehenden Beispiele 3 bis 6 erläutern die Herstellung der als Vorprodukte benötigten Pyrimidine (II) und ein weiteres Beispiel (7) die Herstellung eines 2-Chlor-pyrimidins (IIc).

Beispiel 3

Zu 420 g Trifluoressigsäureanhydrid werden unter Eiskühlung 158 g Pyridin zugegeben. Anschließend wird bei 10 bis 20°C ein Gemisch aus 144 g Ethylvinylether und 158 g Pyridin zugetropft. Man läßt auf 20 bis 25°C erwärmen und rührt bei dieser Temperatur 3 Stunden nach. Dann werden nacheinander 250 ml Wasser, 250 ml konzentrierte Salzsäure und eine Lösung von 120 g Harnstoff in einem Gemisch aus 200 ml Wasser und 400 ml Ethanol zugegeben. Man erwärmt das Reaktionsgemisch zum Sieden und hält es 4 Stunden unter Rückfluß. Nach Abkühlen des Reaktionsgemischs auf 0 bis 5°C wird der Feststoff abgesaugt und im Vakuum bei 50°C getrocknet. Die Ausbeute an 2-Hydroxy-4-trifluormethylpyrimidin beträgt 235 g (72 %).
Schmp. 214 bis 216°C (Z)
$^1$H-NMR ($d_6$-DMSO): 6,91 (d), 8,55 (d), J = 6,6 Hz
IR (KBr): 1652, 1627, 1447, 1319, 1201, 1167, 1112, 1085, 998, 818.

Beispiel 4

In ein Gemisch aus 364 g Trichloracetylchlorid und 316 g Pyridin werden bei 60 bis 65°C 116 g Methylvinylether eingeleitet, dann wird 30 Minuten bei dieser Temperatur gehalten. Man läßt auf 20 bis 25°C abkühlen und gibt dann nacheinander 250 ml Wasser, 250 ml konzentrierte Salzsäure und eine Lösung von 120 g Harnstoff in einem Gemisch aus 200 ml Wasser und 400 ml Ethanol zu. Dann wird wie im Beispiel 1 beschrieben weiterverfahren. Die Ausbeute an 2-Hydroxy-4-trichlormethylpyrimidin beträgt 345 g (81 %).
Schmp. 210 bis 212°C (Z).
$^1$H-NMR ($d_6$-DMSO): 6,97 (d), 8,23 (d); J = 7,0 Hz
IR (KBr): 3017, 1642, 1627, 1544, 1454, 1444, 951, 840, 809, 792.

Beispiel 5

17,2 g Ethylpropenyl-2-ether und 42 g Trifluoressigsäureanhydrid ergeben analog der in Beispiel 1 beschriebenen Verfahrensweise 23,5 g (66 %) 2-Hydroxy-4-trifluormethyl-5-methylpyrimidin.
Schmp.: 100 bis 103°C.

Beispiel 6

Ein Gemisch aus 21,7 g 1,1,1-Trichlor-4-ethoxy-3-buten-2-on, 20 ml Ethanol, 20 ml Wasser, 2 ml konz. Salzsäure und 6,0 g Harnstoff wird 4 Stunden zum Sieden erhitzt nach Abkühlen auf 0 bis 5°C wird der Feststoff abgesaugt. Die Ausbeute an 2-Hydroxy-4-trichlormethylpyrimidin betrug 17,7 g (83 %).
Schmp.: 210 bis 212°C (Z).

Beispiel 7

Eine Lösung von 37,9 g 2-Hydroxy-4-trifluormethylpyrimidin in 400 ml $POCl_3$ wird 2 Stunden auf Rückfluß erwärmt, dann werden 50 g $PCl_5$ zugegeben und bis zum Ende der HCl-Entwicklung (ca. 10 Stunden) unter Rückfluß gehalten.

Anschließend werden unter vermindertem Druck $POCl_3$ und das gebildete 2-Chlor-4-trifluormethylpyrimidin destilliert. Die Ausbeute beträgt 29,6 g (71 %) 2-Chlor-4-trifluormethylpyrimidin.
Sdp.: 105°C/90 mbar.
$^1$H-NMR ($CDCl_3$): 7,75 (d), 9,05 (d), J = 5,0 Hz
IR (KBr): 1576, 1561, 1438, 1352, 1336, 1220, 1181, 1159, 1124, 838, 737, 664 $cm^{-1}$.

Die in der nachstehenden Tabelle aufgeführten Verbindungen wurden ebenfalls auf den in den vorstehenden Beispielen beschriebenen Wegen erhalten, soweit mindestens eine physikalische Angabe zu ihrer Identifizierung vorliegt; andere Verbindungen, die der Formel (I) entsprechen, können auf die gleiche Weise unter entsprechender Abwandlung der Vorschriften nach der jeweils benötigten Menge und (wegen der besten Reaktionsbedingungen) gegebenenfalls nach einem Vorversuch erhalten werden. Sie lassen aufgrund struktureller Ähnlichkeit eine vergleichbare Wirkung erwarten.

Tabelle

| Bsp. Nr. | $R^1$ | $R^2$ | X | Hal | Infrarotabsorption ($cm^{-1}$) oder $^1H$-NMR-Spektren (80 MHz in ($DCl_3$), -Werte) |
|---|---|---|---|---|---|
| 3 | $C_2H_5$ | $O-C_2H_5$ | S | Cl | 1,40 (t, 6H); 4,2 - 4,7 (m, 4H); 7,6 (d, 1H); 9,0 (d, 1H) |
| 4 | $C_2H_5$ | $O-C_2H_5$ | O | F | 1393, 1342, 1301, 1285, 1193, 1158, 1141, 1032, 997, 977 |
| 5 | $CH_3$ | $O-CH_3$ | S | F | 1388, 1341, 1193, 1038, 996, 972 |
| 6 | $C_2H_5$ | $S-n-C_3H_7$ | S | F | 1382, 1340, 1291, 1192, 1158, 1138, 1023, 959 |
| 7 | $C_2H_5$ | $CH_3$ | S | F | 1389, 1341, 1193, 1159, 1139, 1039, 964, 912 |
| 8 | $C_2H_5$ | $C_2H_5$ | S | Cl | 1382, 1306, 1047, 1035, 1019, 990, 964, 875 |
| 9 | $C_2H_5$ | $CH_3$ | S | Cl | 1429, 1381, 1307, 1039, 1021, 969, 911, 898, 873 |
| 10 | $C_2H_5$ | $C_2H_5$ | S | F | 1389, 1340, 1192, 1159, 960 |
| 11 | $CH_3$ | $O-CH_3$ | S | Cl | 3,90 (d, 6H); 7,60 (d, 1H); 9,0 (d, 1H) |
| 12 | $CH_3$ | $O-CH_3$ | O | F | |
| 13 | $C_2H_5$ | $C_6H_5$ | S | F | |
| 14 | $C_2H_5$ | $NH-CH(CH_3)_2$ | O | F | |
| 15 | $C_2H_5$ | $S-sec.-C_4H_9$ | S | F | |
| 16 | $C_2H_5$ | $S-n-C_3H_7$ | O | F | |
| 17 | $CH_3$ | $O-CH_3$ | O | Cl | |
| 18 | $C_2H_5$ | $C_6H_5$ | S | Cl | |
| 19 | $C_2H_5$ | $NH-CH(CH_3)_2$ | O | Cl | |
| 20 | $C_2H_5$ | $S-n-C_3H_7$ | S | Cl | |
| 21 | $C_2H_5$ | $S-sec.-C_4H_9$ | S | Cl | |
| 22 | $C_2H_5$ | $S-n-C_3H_7$ | O | Cl | |

Die Phosphorsäureester der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Die Wirkstoffe können als solche, in Form ihrer Formulierungen oder den daraus bereiteten Anwendungsmöglichkeiten, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Beispiele für Formulierungen sind:

I. 5 Gewichtsteile der Verbindung des Beispiels 1 werden mit 95 Gewichtsteilen feinteiligem Kaolin innig vermischt. Man erhält auf diese Weise ein Stäubemittel, das 3 Gew.% des Wirkstoffs enthält.

II. 30 Gewichtsteile der Verbindung des Beispiels 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung des Beispiels 3 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung des Beispiels 4 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung des Beispiels 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,00001 und 10 %, vorzugsweise zwischen 0,001 und 0,1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,001 bis 10, vorzugsweise 0,1 bis 2,0 kg/ha.

Zu den Wirkstoffen können andere Wirkstoffe verschiedenen Typs, z.B. Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 100 bis 100 : 1 zugemischt werden.

Zur Demonstration der erfindungsgemäßen Wirkung der Stoffe I werden übliche standardisierte Versuche durchgeführt.

Als Vergleichsmittel wurde dabei das aus der DE-OS 22 09 554 bekannte Handelsprodukt Etrimphos

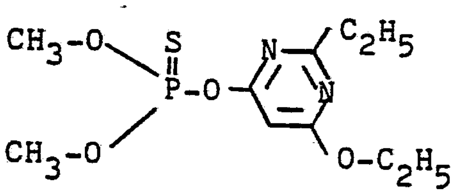

gewählt.

Kontaktwirkung auf Schaben (Blatta orientalis)

Der Boden eines 1 l-Weckglases wird mit der acetonischen Lösung des Wirkstoffes behandelt.

Nach Verdunsten des Lösungsmittels setzt man je Glas 5 adulte Schaben.

Die Mortalitätsrate wird nach 48 Stunden bestimmt.

Ergebnis

| Beispiel | mg | % Mort. |
|---|---|---|
| 1 | 0,1 | 100 |
| 2 | 0,01 | 100 |
| 3 | 0,2 | 100 |
| 4 | 0,02 | 100 |
| 6 | 0,1 | 100 |
| 7 | 0,1 | 100 |
| 8 | 0,1 | 100 |
| 9 | 0,2 | 100 |
| 10 | 0,2 | 100 |

Kornkäfer, Kontakt

Aufgerauhte Glasplatten von 8 x 8 cm Kantenlänge werden mit der azetonischen Wirkstofflösung behandelt.

Nach Verdunsten des Lösungsmittels belegt man die Platten mit 100 Kornkäfer und deckt mit einem Uhrglas (Ø 6 cm) ab. Nach 4 Stunden werden die Käfer in unbehandelte Gefäße überführt. Die Mortalitätsrate wird nach 24 Stunden ermittelt. Dabei wird festgestellt, wieviele Käfer in der Lage waren, nach diesem Zeitpunkt innerhalb von 60 Minuten ein unbehandeltes Pappschälchen (Ø 40 mm, Höhe 10 mm) zu verlassen.

Ergebnis

| Beispiel | mg | % Mort. |
|---|---|---|
| 1 | 0,01 | 100 |
| 9 | 0,01 | 100 |
| | | |
| Vergleichsmittel | 0,02 | 100 |
| | 0,01 | < 60 |

Kontaktwirkung auf Stubenfliegen (Musca domestica) Dauerwirkung behandelter Glasplatten

Aufgerauhte Glasplatten von 15 x 15 cm Seitenlänge werden gleichmäßig mit der acetonischen Wirkstofflösung behandelt. Nach dem Verdunsten des Lösungsmittels werden je 10 4-Tage alte Stubenfliegen unter einer Petrischale (Ø 10 cm) auf die Glasplatten gesetzt. Die Mortalität nach 4 Stunden gilt als Richtwert im Versuch.

Die Prüfungen werden in Tagesabständen wiederholt, bis die behandelten Platten keine Wirkung mehr zeigen. Die Versuchstemperatur beträgt 20 - 22°C.

Ergebnis

| Beispiel | mg | Tage | % Mort. |
|---|---|---|---|
| 1 | 1 | 8 | 100 |
| 4 | 1 | 8 | 85 |
| 6 | 1 | 4 | 100 |
| 8 | 1 | 8 | 100 |
| 9 | 1 | 8 | 100 |
| Vergleichsmittel | 1 | 2 | 100 |
| | | 4 | < 60 |

Zuchtversuch mit Stubenfliegen-Larven (Musca domestica)

Je 4,5 ml Magermilch füllt man in 50 ml Pennicillingläser und versetzt sie darauf mit 0,5 ml der wäßrigen Wirkstoffaufbereitung. Nach kurzem Mischen fügt man ein Wattebällchen (Firma Hartmann, Maintal) dazu und belegt dieses mit ca. 50 Ei-Larven der Stubenfliege.

Die Gläser lagert man abgedeckt bei Raumtemperatur und beurteilt die Entwicklung nach 7 Tagen.

Ergebnis

| Beispiel | ppm | |
|---|---|---|
| 2 | 0,2 | totale Hemmung |
| 3 | 1,0 | ca. 90 % Hemmung |
| 4 | 0,1 | ca. 90 % Hemmung |
| 8 | 1,0 | totale Hemmung |
| Vergleichsmittel | 2,0 | totale Hemmung |

Kontaktwirkung auf Baumwollwanze (Dysdercus intermedius)

Petrischalen von 10 cm Durchmesser werden mit 1 ml der azetonischen Wirkstofflösung ausgekleidet.

Nach Verdunsten des Lösungsmittels besetzt man die Schalen mit je 20 Larven des vorletzten Stadiums und registriert die Wirkung nach 24 Stunden.

Ergebnis

| Beispiel | mg | % Mort. |
|---|---|---|
| 2 | 0,002 | 80 |
| 4 | 0,002 | 100 |
| 10 | 0,002 | 80 |
| Vergleichsmittel | 0,005 | 80 |

Plutella maculipennis; Kohlschaben-Raupen; Fraß- und Kontaktwirkung

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Wirkung.

Ergebnis

| Beispiel | % | % Mort. |
|---|---|---|
| 1 | 0,01 | 100 |
| 2 | 0,001 | ca. 80 |
| 3 | 0,01 | 100 |
| 4 | 0,002 | 100 |
| 6 | 0,01 | 100 |
| 7 | 0,001 | ca. 80 |
| 8 | 0,004 | 100 |
| 9 | 0,01 | 100 |
| 10 | 0,002 | 100 |

Patentansprüche

1. Pyrimidylphosphorsäureester der Formel I

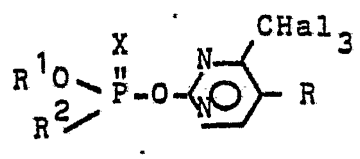

(I),

in der

R Wasserstoff oder Niederalkyl, das gegebenenfalls durch Halogen, Niederalkoxy, -Alkylthio, Aryloxy oder Aryl substituiert ist,

$R^1$ Niederalkyl,

$R^2$ Niederalkoxy- oder -Alkylthio, Niederalkyl, Phenyl, Niederalkylamino- oder Niederdialkylamino

X Sauerstoff oder Schwefel und

Hal Fluor oder Chlor bedeuten.

2. Verfahren zur Herstellung von Estern der Formel I, dadurch gekennzeichnet, daß man ein entsprechendes 2-Hydroxy-4-trihalogenmethylpyrimidin(II) bzw. dessen Salz

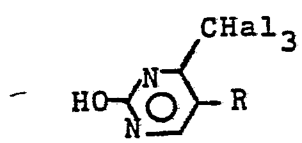

(II),

mit einem entsprechenden Phosphorsäureesterhalogenid(III)

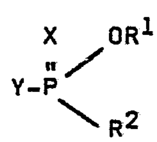

(III),

in dem

Y für Halogen steht, umsetzt.

3. Schädlingsbekämpfungsmittel, enthaltend einen Ester (I) gemäß Anspruch 1.

4. Schädlingsbekämpfungsmittel, enthaltend einen festen oder flüssigen Trägerstoff und mindestens einen Ester der Formel I gemäß Anspruch 1.

5. Verwendung von Estern der Formel I gemäß Anspruch 1 zur Bekämpfung von Schädlingen.

6. Verfahren zur Bekämpfung von Schädlingen, dadurch gekennzeichnet, daß man eine wirksame Menge des Esters gemäß Anspruch 1 auf Schädlinge bzw. deren Lebensraum einwirken läßt.

7. 2-Hydroxypyrimidine der Formel

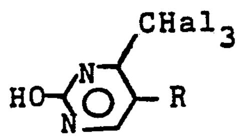

(II),

in der

Hal Fluor oder Chlor und

R Wasserstoff oder Niederalkyl, das gegebenenfalls durch Halogen, Niederalkoxy, -Alkylthio, Aryloxy oder Aryl substituiert ist,

bedeuten.

8. Verfahren zur Herstellung von 2-Hydroxypyrimidinen (II), dadurch gekennzeichnet, daß man ein Trihalogenmethylcarbonsäureanhydrid oder ein Trihalogenmethylcarbonsäurehalogenid mit einem Enolether in an sich bekannter Weise umsetzt und auf das Umsetzungsprodukt Harnstoff einwirken läßt.

9. Verwendung der 2-Hydroxypyrimidine (II) zur Herstellung entsprechender der 2-Chlorpyrimidine.

**0163280**

Europäisches Patentamt

# EUROPÄISCHER RECHERCHENBERICHT

Nummer der Anmeldung

EP 85 10 6466

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
| A | EP-A-0 003 098 (BAYER)<br>--- | 1 | C 07 F 9/65<br>C 07 D 239/36<br>A 01 N 57/16<br>A 01 N 57/24 |
| A | CHEMICAL ABSTRACTS, Band 73, Nr. 11, 14. September 1970, Columbus, Ohio, USA; J. JONAK "Alkylations of heterocyclic ambident anions. III. 4-Hydroxypyrimidines", Seite 266, Abstraktnr. 55351c & J. Org. Chem. 1970, Band 35 Nr. 8, Seiten 2512-2 516<br>--- | 7 | |
| A | DE-B-2 120 247 (LONZA)<br>--- | 7 | |
| A,D | DE-A-3 003 337 (SUMITOMO)<br>----- | 1 | |
| | | | RECHERCHIERTE SACHGEBIETE (Int. Cl.4) |
| | | | C 07 F 9/65<br>C 07 D 239/36 |

Der vorliegende Recherchenbericht wurde für alle Patentansprüche erstellt.

| Recherchenort | Abschlußdatum der Recherche | Prüfer |
|---|---|---|
| BERLIN | 22-08-1985 | KAPTEYN H G |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03 82